# EUROPÄISCHE PATENTSCHRIFT

(11) **EP 1 510 173 B1**
(45) Veröffentlichungstag und Bekanntmachung des Hinweises auf die Patenterteilung: **05.04.2017**
(21) Anmeldenummer: 04090233.0
(22) Anmeldetag: 14.06.2004
(51) Int. Cl.: A61B 5/053, A61N 1/365

(54) **Intrakardiale Impedanzmessanordnung**
Intracardial impedance measuring device
Appareil de mesure d'impédances intracardiaques

(30) Priorität: 01.09.2003 DE 10340894; 16.12.2003 DE 10361143
(43) Veröffentlichungstag der Anmeldung: 02.03.2005
(73) Patentinhaber: BIOTRONIK SE & Co. KG, 12359 Berlin (DE)
(72) Erfinder: Czygan, Gerald, Dr. Ing., 91054 Buckenhof (DE); Lippert, Michael, Dr., 91522 Ansbach (DE)
(74) Vertreter: Galander, Marcus

(56) Entgegenhaltungen:
- WO-A-00/78391
- WO-A-94/06513
- WO-A-03/051457
- WO-A-2004/050177
- US-A- 5 154 171
- US-A1- 2001 012 953
- US-A1- 2002 002 389

## Beschreibung

Die Erfindung betrifft ein Implantat mit Elektrodenleitungsanschlüssen zum Anschluss intrakardialer und/oder epikardialer Elektrodenleitungen, wobei die Elektrodenleitungsanschlüsse zusammen wenigstens drei elektrische Kontakte aufweisen, von denen wenigstens einer einer rechtsventrikulären Elektrode und ein anderer einer linksventrikulären Elektrode zugeordnet ist. Das Implantat weist eine Impedanzmesseinheit auf, die eine Strom- oder Spannungsquelle und eine Messeinrichtung für eine entsprechende Spannungs- bzw. Strommessung aufweist, die derart mit den elektrischen Kontakten und gegebenenfalls einer Gehäuseelektrode des Implantats verbunden sind, dass sich eine tri- oder quadrupolare Impedanzmessanordnung ergibt.

Derartige Implantate sind als Herzschrittmacher, beispielsweise aus der WO 03/51457, der WO 00/78391 oder der US 2001/0012953, grundsätzlich bekannt.

WO 2004/050177 A1 ist Stand der Technik unter Art. 54 (3) EPÜ und beschreibt einen Herzschrittmacher mit einem Stimulationsimpulsgenerator für eine biventrikuläre Stimulation des Herzens, wobei der Stimulationsimpulsgenerator mit rechtsventrikulären Elektroden für die Stimulation eines rechten Ventrikels des Herzens und mit linksventrikulären Elektroden für die Stimulation eines linken Ventrikels des Herzens zu verbinden ist. Die Elektroden sind mit einer Impedanzbestimmungseinheit derart verbunden, dass sich eine tri- oder quadrupolare Impedanzmessanordnunng ergibt. es werden folgende Werte ermittelt: ein sich aus einem zeitlichen Verlauf der Impedanzmesswerte ergebendes Impedanzsignal, ein Minimum der Impedanzmesswerte Zmin innerhalb eines ersten Zeitfensters Tmin und ein Maximum der Impedanzmesswerte ZES innerhalb eines zweiten Zeitfensters TES, wobei das erste Zeitfenster Tmin und das zweite Zeitfenster TES kürzer sind als ein jeweiliges Herzzyklusintervall und relativ zu einem ventrikulären Ereignis in einem Herzzyklus orientiert sind. Die Auswerteeinheit bestimmt das Maximum der 2. Ableitung Z"max und den Zeitpunkt davon im Herzzyklusintervall T"max und bestimmt somit aus der zweiten Ableitung des Impedanzsignals eine eine Kontraktilität eines Herzens entsprechende Kontraktilitätsgröße.

Dennoch besteht nach wie vor ein Bedürfnis nach einem Implantat, welches eine verbesserte Erfassung und Nutzung von Impedanzwerten ermöglicht.

Erfindungsgemäß wird dies mit einem Implantat gemäß Anspruch 1 erreicht.

Die abhängigen Ansprüche definieren bevorzugte Ausführungsformen der Erfindung.

Die hierin beschriebenen Aspekte, Ausführungsvarianten, Ausführungsformen und Ausführungsbeispiele, die nicht unter den Wortlaut der Ansprüche fallen sind nicht Teil der Erfindung.

Ein derartiges Implantat erlaubt es in vorteilhafter Weise, mittels Impedanzmessung hämodynamische Größen, wie beispielsweise das Schlagvolumen, zu ermitteln.

Hämodynamische Größen werden augenblicklich mit Hilfe von Echokardiographie, über Thoraximpedanzkardiographie, mit Hilfe von Thermodilutionskathetern oder mit invasiven Druckmessungen während elektrophysiologischer Untersuchungen ermittelt. Diese Verfahren verlangen einen hohen klinischen Aufwand. Zu Forschungszwecken werden gelegentlich Schrittmacher implantiert, die ventrikuläre Druckmessungen mit Hilfe eines Sensors durchführen, der in eine Stimulationselektrodenleitung integriert ist. Dies bedeutet, dass spezielle Elektrodenleitungen für ein derartiges Gerät erforderlich sind.

Hämodynamische Größen des Blutkreislaufs, insbesondere das Schlagvolumen (SV), das enddiastolische Volumen (EDV), das endsystolische Volumen (ESV) oder die Kontraktilität des Herzens sowie die Abmessungen des Ventrikels liefern wichtige Informationen über den Zustand des kardiovaskularen Systems. Die Elektrotherapie des Herzens mit Hilfe von Implantaten kann durch einen Sensor, der hämodynamische und geometrische Größen erfasst, verbessert werden.

Kontinuierliches Überwachen von Patienten kann erzielt werden, indem hämodynamische oder geometrische Daten zum Zwecke des Homemonitorings telemetrisch übermittelt werden. Insbesondere für Patienten, die an einem Herzversagen leiden, ist die Beobachtung des hämodynamischen Zustands wesentlich, insbesondere die Beobachtung des Fortschreitens (oder der Besserung) der Erkrankung oder die Überwachung des Patientenzustands im Rahmen einer Resynchronisations- oder einer Medikamententherapie.

Erfindungsgemäß umfasst die intrakardiale Impedanzmessvorrichtung ein elektrotherapeutisches Implantat, beispielsweise einen implantierbaren Schrittmacher oder Kardioverter/Defibrillator, der eine Messeinrichtung zum Bestimmen einer intrakardialen Impedanz oder eines intrakardialen Impedanzverlaufs (Impedanzsignals) aufweist. Die Elektroden des Implantats sind vorzugsweise in drei oder vier Kammern des Herzens angeordnet, so dass die Anordnung auch für die Mehrkammerstimulation und/oder Defibrillation geeignet ist. Zumindest eine bipolare Elektrode sollte geeignet sein, im rechten Ventrikel (RV) angeordnet zu werden und eine zweite bipolare Elektrode in der Nähe des linken Ventrikels (LV), und zwar entweder durch Anordnung in einer vom Koronarsinus abzweigenden Lateralvene oder am Epikard. Die linksventrikuläre Elektrode ist somit eine Koronarsinus-Elektrode oder eine epikardiale Elektrode.

Das erste und das zweite Zeitfenster sind kürzer als ein jeweiliges Herzzyklus-Intervall und relativ zu einem ventrikulären Ereignis in einem Herzzyklus orientiert. Erfindungsgemäß beginnt ein erstes Zeitfenster zu einem ersten Anfangszeitpunkt (X1) vor dem jeweiligen ventrikulären Ereignis und endet zu einem ersten Endzeitpunkt (X2) nach diesem ventrikulären Ereignis, während ein zweites Zeitintervall zu einem zweiten Anfangszeitpunkt (Y1) nach jenem ventrikulären Ereignis beginnt und zu einem zweiten Endzeitpunkt (Y2) nach dem ventrikulären Ereignis endet.

Die Auswerteeinheit ist vorzugsweise ausgebildet, aus einer Differenz der enddiastolischen Impedanz (EDZ) und der endsystolischen Impedanz (ESZ) eine ein Schlagvolumen repräsentierende Schlagimpedanz (SZ) zu ermitteln.

Alternativ oder zusätzlich ist die Auswerteeinheit ausgebildet, aus der Schlagimpedanz (SZ) und der enddiastolischen Impedanz (EDZ) eine einen Auswurfanteil (EF = Ejektionsfraktion) repräsentierende EF-Größe zu ermitteln. Anstelle der enddiastolischen Impedanz EDZ und der endsystolischen Impedanz ESZ können auch deren Kehrwerte ausgewertet werden, nämlich die enddiastolische Leitfähigkeit EDC (EDC = 1/EDZ) und die endsystolische Leitfähigkeit ESC (ESC = 1/ESZ). Der Auswurfanteil EF = SV/EDV ist dann annähernd proportional zu (EDC-ESC)/EDC.

Erfindungsgemäß ist die Auswerteeinheit ausgebildet ein sich aus einem zeitlichen Verlauf der Impedanzmesswerte ergebendes Impedanzsignal zu bestimmen und aus der ersten oder zweiten Ableitung des Impedanzsignals eine eine Kontraktilität eines Herzens repräsentierende Kontraktilitätsgröße zu ermitteln.

Die zuletzt genannten drei hämodynamischen Größen sind für den Arzt besonders interessant. Grundsätzlich ist jede Art von Implantat vorteilhaft, mit dem sich interessante hämodynamische Größen mittels Impedanzmessung ermitteln lassen.

Das Implantat weist vorzugsweise einen Speicher für einen oder mehrere der Werte für die Schlagimpedanz, die EF-Größe oder die Kontraktilität auf. In diesem Zusammenhang ist die Auswerteeinheit vorzugsweise ausgebildet, die Werte für die Schlagimpedanz, die enddiastolische Impedanz (EDZ), die EF-Größe und/oder die Kontraktilität zu regelmäßig wiederkehrenden Speicherzeitpunkten zu speichern.

Besonders interessant ist es, wenn die Auswerteeinheit ausgebildet ist, für einen Zeitraum zwischen zwei aufeinander folgenden Speicherzeitpunkten Mittelwerte für die Schlagimpedanz, die enddiastolische Impedanz (EDZ), die EF-Größe und/oder die Kontraktilität zu bilden und einen oder mehrere dieser Mittelwerte zu speichern.

Darüber hinaus ist die Auswerteeinheit vorzugsweise dazu ausgebildet, aus der zeitlichen Entwicklung der Schlagimpedanz, der enddiastolischen Impedanz (EDZ), der EF-Größe oder der Kontraktilität einen jeweiligen Trendwert für einen oder mehrere dieser Größen zu ermitteln. Auch diese Trendwerte werden vorzugsweise zu einem jeweiligen Speicherzeitpunkt durch die Auswerteeinheit in dem Speicher gespeichert.

Außerdem weist das Implantat vorzugsweise eine Telemetrieeinheit auf, die wenigstens einen Telemetriesender umfasst und mit dem Speicher verbunden sowie ausgestaltet ist, auf eine Abfrage hin oder zu regelmäßigen Sendezeitpunkten Werte für Schlagimpedanz, enddiastolische Impedanz (EDZ), EF-Größe, Kontraktilität oder einen oder mehrere Mittel- oder Trendwerte an ein externes Gerät zu senden.

Im Übrigen weist das Implantat vorzugsweise alle bekannten und vorteilhaften Merkmale eines Herzschrittmachers, Cardioverters und/oder Defibrillators auf. Zu diesen Merkmalen zählt insbesondere wenigstens eine ventrikuläre oder eine artriale Stimulationseinheit sowie eine Steuereinheit, mit der sich Stimulationsgrößen wie beispielsweise die Stimulationsimpulsstärke, eine Stimulationsfrequenz oder Ähnliche einstellen lassen. Ein derartiges Implantat ist vorzugsweise als ratenvariabler Herzschrittmacher ausgebildet, der einen Sensor für den physiologischen Bedarf eines Patienten aufweist, mit dessen Hilfe die Stimulationsrate an den physiologischen Bedarf eines Patienten durch das Implantat automatisch anzupassen ist.

Weitere bevorzugte Ausführungsvarianten ergeben sich beispielsweise aus der nachfolgenden Beschreibung bevorzugter Ausführungsbeispiele.

In einer bevorzugten Ausführungsform weist das Implantat eine Auswerteeinheit auf, die zum Bestimmen der Änderungen des linksventrikulären Durchmessers ausgebildet ist. Optional kann die Auswerteeinheit auch ausgebildet sein, hämodynamische Größen anderer Herzkammern via Impedanzmessung zu ermitteln. Ein wichtiger Vorteil der Erfindung ist es, dass die intrakardiale Impedanzmessanordnung für die Impedanzmessung lediglich übliche Stimulations- oder Defibrillationselektrodenleitungen benötigt.

Es hat sich herausgestellt, dass eine quadrupolare Impedanzmessanordnung mit zwei Elektroden im rechten Ventrikel zur Stromeinspeisung und zwei weiteren Elektroden im Koronarsinus, die dem linken Ventrikel zugeordnet sind, für die Messung einer aus dem eingespeisten Strom resultierenden Spannung besonders vorteilhaft und störunanfällig ist. Dementsprechend ist ein Implantat besonders bevorzugt, welches eine quadrupolare Impedanzmessanordnung aufweist, die für den Anschluss an zwei rechtsventrikuläre Elektroden zur Stromeinspeisung und zwei linksventrikuläre, in einer vom Koronarsinus abzweigenden Lateralvene angeordnete Elektroden zur Messung der aus dem eingespeisten Strom resultierenden Spannung ausgebildet ist

Die Erfindung soll nun anhand von Ausführungsbeispielen mit Hilfe der beigefügten Figuren näher erläutert werden. Von den Figuren zeigt:
- Fig. 1a und 1b:: zwei schematische Darstellungen zwei sehr ähnlicher bevorzugter Ausführungsvarianten eines Implantats;
- Fig. 2a und 2b:: zwei bevorzugte Messkonfigurationen für die Bestimmung des linken Ventrikels
- Fig. 3:: eine alternative Impedanzmesskonfiguration für den linken Ventrikel
- Fig. 4:: eine weitere Alternative einer Impedanzmessanordnung für den linken Ventrikel
- Fig. 5:: ein intrakardiales Elektrokardiogramm, eine Darstellung des Ventrikelvolumens sowie eine Darstellung der daraus resultierenden Impedanz in zeitlicher Zuordnung zueinander;
- Fig. 6:: ein Beispiel für ein gemessenes Impedanzsignal.

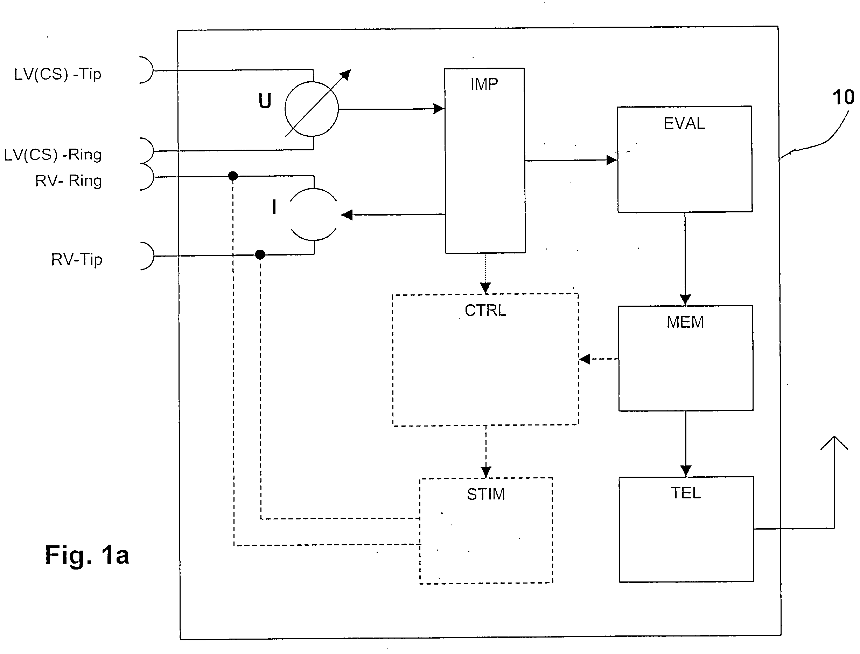

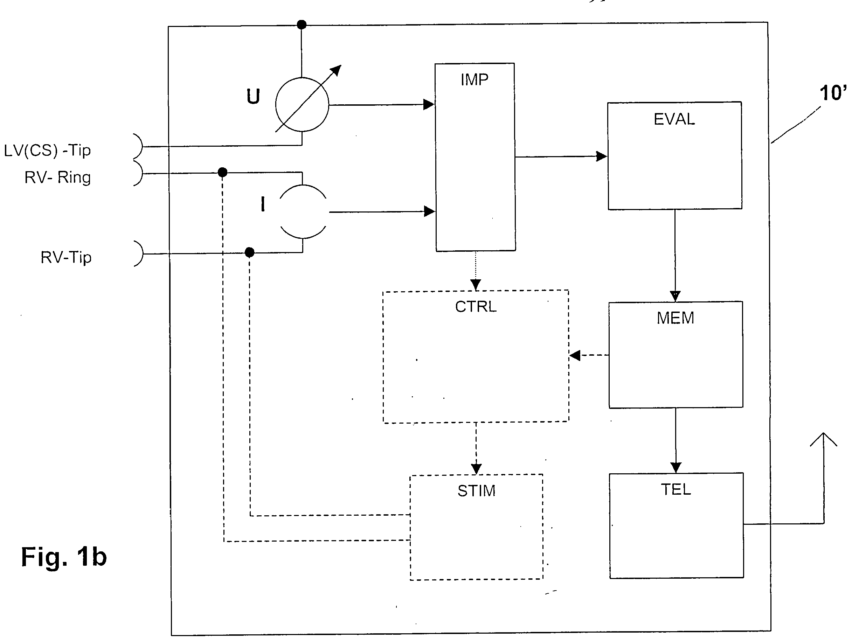

Die Figuren 1a und 1b zeigen in schematischer Darstellung ein Implantat 10 bzw. 10' mit einer Impedanzmessanordnung, die eine Stromquelle I und eine Spannungsmesseinheit U sowie Impedanzbestimmungseinheit IMP aufweist.

Die Spannungsmesseinheit U ist bei der Ausführungsvariante in Figur 1a mit einer in einer vom Koronarsinus abzweigenden Lateralvene angeordneten, linksventrikulären Spitzenelektrode sowie einer ebenfalls in einer vom Koronarsinus abzweigenden Lateralvene angeordneten, linksventrikulären Ringelektrode verbunden. Die Stromeinspeisungseinheit I ist mit einer rechtsventrikuläre Tip- und mit einer rechtsventrikulären Ringelektroden - oder genauer gesagt mit Kontakten für den Anschluss dieser Elektroden - verbunden.

In der Ausführungsvariante gemäß Figur 1b ist die Spannungsmesseinheit U auf einerseits wie bei Fig. 1a mit linksventrikulären Tip-Elektrode und abweichend von Fig. 1a andererseits mit dem Implantatsgehäuse als vierte Elektrode verbunden.

Die Impedanzbestimmungseinheit IMP ist für die Impedanzbestimmung sowohl mit der Stromeinspeisungseinheit I als auch mit der Spannungsmesseinheit U verbunden. Der jeweils ermittelte Impedanzwert wird seitens der Impedanzbestimmungseinheit IMP an eine Auswerteeinheit EVAL weitergegeben. Die Auswerteeinheit EVAL bestimmt auf die nachfolgend beschriebene Art und Weise aus den von der Impedanzbestimmungseinheit IMP ermittelten Werten eine enddiastolische Impedanz EDZ und eine endsystolische Impedanz ESZ.

Außerdem leitet die Auswerteeinheit EVAL aus diesen Werten eine Schlagimpedanz SZ als Differenz aus endsystolischer Impedanz und enddiastolischer Impedanz (SZ = ESZ - EDZ) ab. Dies geschieht in Verbindung mit einem Plausibilitätscheck, währenddessen geprüft wird, ob die enddiastolische Impedanz (EDZ) kleiner ist als die endsystolische Impedanz (ESZ).

Weitere von der Auswerteeinheit EVAL ermittelte Werte sind für jeden Herzzyklus einen Auswurfanteil (Ejection Fraction = EF), der aus der Schlagimpedanz und der enddiastolischen Impedanz (EF ∼ SZ * EDZ, da EF = SV/EDV und SV-SZ sowie EDV∼1/EDZ) oder der enddiastolischen Leitfähigkeit (EDC) und der endsystolischen Leitfähigkeit (ESC) zu bilden ist, sowie eine eine Kontraktilität eines Herzens repräsentierende Kontraktilitätsgröße. All diese Werte werden von der Auswerteeinheit EVAL in einem Speicher MEM gespeichert, und zwar vorzugsweise zu regelmäßig wiederkehrenden Speicherzeitpunkten.

Die Auswerteeinheit EVAL ist weiterhin dazu ausgebildet, Mittelwerte für die Schlagimpedanz, die EF-Größe oder die Kontraktilität für einen jeweiligen zwischen zwei Speicherzeitpunkten liegenden Zeitraum zu bilden und diese Mittelwerte ebenfalls in dem Speicher zu speichern.

Weiterhin ist die Auswerteeinheit EVAL ausgebildet, Trends für die von der Auswerteeinheit EVAL ermittelten Größen zu bestimmen und entsprechende Trendwerte in dem Speicher MEM zu speichern.

Der Speicher MEM ist ausgangsseitig mit einer Telemetrieeinheit TEL verbunden, die so ausgebildet ist, dass die in dem Speicher jeweils gespeicherten Werte zu einem regelmäßig wiederkehrenden Sendezeitpunkt von der Telemetrieeinheit mittels einer der Telemetrieeinheit zugeordneten Sendeeinheit derart ausgesandt werden, dass die entsprechenden Werte von einem externen Gerät empfangen und beispielsweise an ein Servicecenter, einen Arzt oder dergleichen weitergeleitet werden können.

Gestrichelt eingezeichnet sind Beispiele für übliche Bestandteile eines derartigen Implantates nämlich eine Steuereinheit CTRL, die mit der Impedanzbestimmungseinheit IMP verbunden ist und eine Stimulationseinheit SDIM steuert. Im dargestellten Beispiel ist die Stimulationseinheit SDIM eine rechtsventrikuläre Stimulationseinheit und somit mit dem Anschluss für die rechtsventrikuläre Ring- und die rechtsventrikuläre Tipelektrode verbunden.

Zur Impedanzmessung injiziert die Impedanzmesseinheit einen unterschwelligen Strom zwischen zwei Elektroden der an das Implantat angeschlossenen Elektrodenleitungen und/oder das Implantatsgehäuse. Der Strom hat die Form biphasischer Impulse mit konstanter Amplitude. Der durch den Strom hervorgerufene Spannungsabfall (die Spannung) wird über ein anderes Elektrodenpaar der zur Verfügung stehenden Elektroden gemessen. Die gemessene Spannung ist proportional zur Impedanz des Gewebes, welches sich im Messbereich befindet. In einer alternativen Ausführungsvariante können die strominjizierenden Elektroden und die Elektroden zur Spannungsmessung dieselben Elektroden sein.

Die gemessene Spannung wird zunächst verstärkt und mittels einer programmierbaren Filteranordnung gefiltert, bevor sie analog/digital gewandelt wird. Der programmierbare Filter kann ein Tiefpassfilter sein, ein Hochpassfilter oder ein Bandpassfilter. In dem nachfolgend beschriebenen Ausführungsbeispiel ist der Filter ein Tiefpassfilter. Die Messelektrodenanordnung ist wie zuvor erläutert vorzugsweise quadrupolar, um insbesondere Durchmesseränderungen des Ventrikels mittels Impedanzmessung zu erfassen. Dementsprechend wird der Strom über zwei Elektroden injiziert und die Spannung über zwei andere, von den strominjizierenden Elektroden verschiedene Elektroden gemessen.

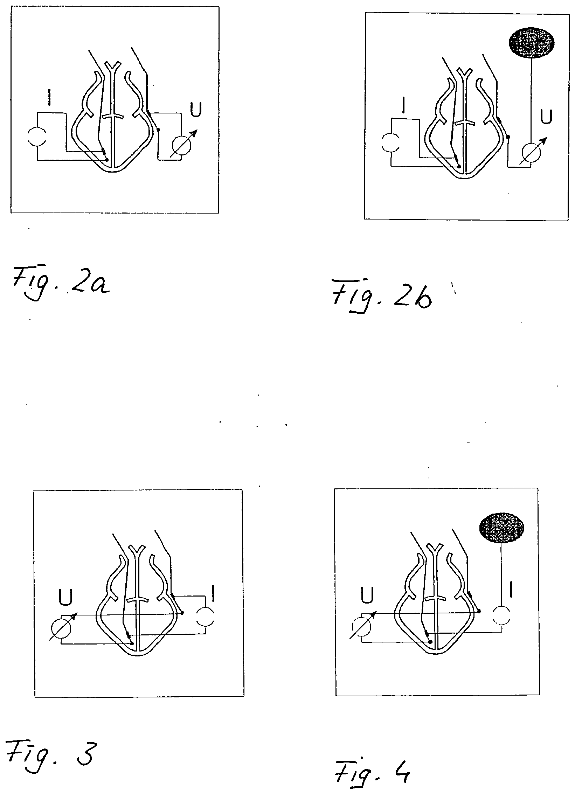

Von den verschiedenen möglichen Konfigurationen sind zwei Konfigurationen für die Messung im linken Ventrikel besonders bevorzugt:
1. Der Strom für die Impedanzmessung wird zwischen einer rechtsventrikulären Spitzenelektrode und einer rechtsventrikulären Ringelektrode eingespeist. Die daraus resultierende Spannung wird zwischen einer linksventrikulären Tipelektrode und einer linksventrikulären Ringelektrode gemessen. Die linksventrikulären Elektroden können sich dabei in einer vom Koronarsinus abzweigenden Lateralvene befinden oder epikardial angeordnet sein. (Fig. 2a)
2. Alternativ ist eine Stromeinspeisung ebenfalls über die rechtsventrikuläre Spitzenelektrode und die rechtsventrikuläre Ringelektrode vorgesehen, jedoch die Spannungsmessung zwischen einer linksventrikulären Spitzenelektrode und dem Implantatsgehäuse. (Fig. 2b)

Das Impedanzsignal, das mit diesen Konfigurationen gemessen wird, hängt kubisch vom Abstand zwischen den beiden Elektrodenleitungen ab. Für ein Dipolfeld in einem homogenen Medium gilt: 1/Z ≈ d³, wobei Z die Impedanz ist und d der Abstand zwischen den Elektrodenleitungen. Dementsprechend ist der Kehrwert der Impedanz ein indirektes Maß für das linksventrikuläre Volumen, weil das linksventrikuläre Volumen annähernd proportional zur dritten Potenz des linksventrikulären Durchmessers ist. Dem liegt die Annahme zgrunde, dass der Abstand a der beiden einen Elektroden eines stromeinspeisenden Dipols zueinander sehr viel kleiner ist, als der Abstand d der spannungsmessenden Elektrode(n) von dem stromeinspeisenden Dipol.

Zwei alternative Elektrodenkonfigurationen umfassen:
3. Eine Stromeinspeisung zwischen rechtsventrikulärer Ringelektrode und linksventrikulärer Ringelektrode und eine Spannungsmessung zwischen rechtsventrikulärer Spitzenelektrode und linksventrikulärer Spitzenelektrode (Fig. 3); oder
4. Stromeinspeisung zwischen rechtsventrikulärer Ringelektrode und Implantatsgehäuse sowie Spannungsmessung zwischen rechtsventrikulärer Spitzenelektrode und linksventrikulärer Spitzenelektrode (Fig. 4).

Bei diesen Anordnungen entspricht der Kehrwert der Impedanz dem Abstand zwischen den Elektrodenleitungen, falls - wie in den Konfigurationen 3 und 4 vorgesehen - a sehr viel kleiner ist als d.

Die zuvor genannten Konfigurationen 2 (Fig. 2b) und 4 (Fig. 4) bieten sich dann an, wenn nur eine unipolare linksventrikuläre Elektrodenleitung zur Verfügung steht.

Die Auswertung des Impedanzsignals erfolgt durch die Auswerteeinheit im Implantat, die mit der Messeinheit verbunden ist. Die Auswerteeinheit ist ausgebildet, aus dem gemessenen Impedanzsignal Parameter abzuleiten, insbesondere den zeitlichen Verlauf der Impedanz Z f(t), der vom enddiastolischen und endsystolischen Durchmesser des Ventrikels abhängt und damit vom enddiastolischen Volumen (EDV), vom endsystolischen Volumen (ESV) und vom Schlagvolumen (SV) der entsprechenden Kammer. Die Auswertung gilt Relativwerten dieser Größen und nicht deren Absolutwerten.

Der der Bestimmung des relativen Volumens zu Grunde liegende, allgemeine Gedanke fußt auf den Unterschieden in den Abständen zwischen rechtsventrikulärer und linksventrikulärer Elektrodenleitungen während eines Kontraktionszykluses. Der Ventrikel dehnt sich während der Diastole aus und erreicht seine maximalen Durchmesser am Ende dieser Phase. Dementsprechend ist die Impedanz am Ende der Diastole minimal, weil die Distanz zwischen den beiden Elektrodenleitungen maximal ist. Die enddiastolische Impedanz wird im folgenden auch mit EDZ bezeichnet. Auf der anderen Seite ist die Impedanz am Ende der Systole maximal, weil der Abstand zwischen den Elektrodenleitungen wegen der Kontraktion des Ventrikels minimal ist. Die dementsprechende endsystolische Impedanz wird im folgenden auch als ESZ bezeichnet. Als Schlagimpedanz SZ wird die Differenz zwischen endsystolischer Impedanz und enddiastolischer Impedanz bezeichnet: SZ = ESZ - EDZ. Die Schlagimpedanz SZ ist proportional zum Schlagvolumen SV des Ventrikels. Außerdem tragen die verschiedenen Leitfähigkeiten des Blutes und des umgebenden Myokards zur Änderung des Impedanzsignals bei. Die Leitfähigkeit des Blutes ist etwa um den Faktor 1,5 bis 2 höher als die Leitfähigkeit des Myokards. Die Blutmenge im Messbereich ist während der enddiastolischen Phase maximal und während der endsystolischen Phase minimal. Dieser Effekt trägt zu den Impedanzänderungen bei, die durch den alternierenden ventrikulären Durchmesser verursacht sind.

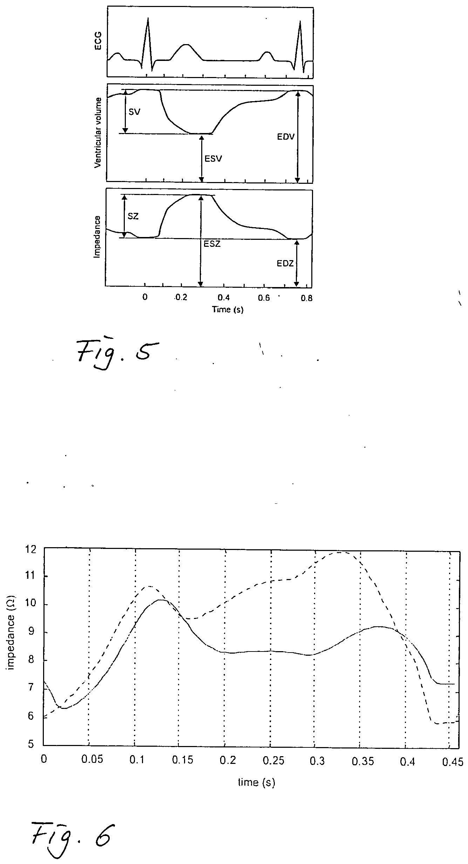

In Figur 5 ist der ideale Verlauf des Impedanzsignals dargestellt. In der Praxis wird das Signal vom idealen Verlauf abweichen, weil es durch andere Einflüsse gestört wird. Ein Verfahren zur Verarbeitung realer Impedanzsignale zum Extrahieren der relevanten Parameter umfasst die folgenden Auswertungsschritte:
1. Die Messung wird durch ein eindeutiges Signal ausgelöst, welches den Beginn eines Herzzyklusses charakterisiert. Ein Herzzyklus beginnt mit einer ventrikulären Kontraktion, d.h. mit einem ventrikulären Event in einem intrakardialen EKG und endet mit dem nächstfolgenden Ereignis. Das auslösende Signal kann entweder direkt aus dem ventrikulären intrakardialen Elektrokardiogramm abgeleitet werden oder alternativ oder zusätzlich durch den Markerkanal des Implantats gewonnen werden. Als ventrikuläres Ereignis oder ventrikulärer Event wird hierbei ein elektrisches Signal verstanden, das mit einer ventrikulären Kontraktion einhergeht oder diese auslöst. Dies kann ein Stimulationsimpuls des Schrittmachers sein oder ein intrinsisches, natürliches Ereignis. Ein solches intrinsisches oder natürliches Ereignis ist bekanntermaßen durch den QRS-Komplex in einem intrakardialen Elektrokardiogramm gegeben.
2. Die Impedanzsignale von n aufeinanderfolgenden Herzzyklen werden ermittelt, um Rauschen und Atmungsartefakte (durch die Respiration bedingte Signalanteile) zu eliminieren.
3. Die enddiastolische Impedanz wird als die minimale Impedanz Z des gemittelten Impedanzsignals innerhalb eines vorgegebenen Zeitfensters bestimmt, welches x₁ ms (ms = Millisekunden) vor dem ventrikulären Ereignis startet und x₂ ms nach dem ventrikulären Ereignis endet. x₁ kann dabei negativ sein, so dass das Zeitfenster auch nach dem ventrikulären Ereignis starten kann. Die endsystolische Impedanz wird als der Maximalwert der Impedanz Z des gemittelten Impedanzsignals während eines zweiten Zeitfensters zwischen y₁ ms und y₂ ms nach einem ventrikulären Ereignis bestimmt. y₁ und y₂ können negative Werte haben, d.h. dass das Zeitfenster auch relativ zum nächstfolgenden ventrikulären Ereignis bestimmt sein kann.
4. Die Schlagimpedanz SZ wird aus der enddiastolischen Impedanz EDZ und der endsystolischen Impedanz ESZ berechnet. Das Vorzeichen der Schlagimpedanz SZ wird einer Plausibilitätsprüfung unterzogen, d.h. die enddiastolische Impedanz muss kleiner sein als die endsystolische Impedanz. Wenn dies nicht der Fall ist, beispielsweise aufgrund umgekehrter Messpolarität, wird das Vorzeichen berichtigt.

In Figur 6 ist ein Beispiel für ein gemessenes Impedanzsignal dargestellt. Auf der Zeitskala entsprechen 0 ms hier dem Punkt 50 ms vor der R-Zacke des rechtsventrikulären intrakardialen Elektrokardiogramms im Falle eines Stimulus. Das erste Maximum tritt nicht am Ende der Systole auf, sondern ist durch andere Einflüsse bedingt. Es sollte daher nicht innerhalb des systolischen Zeitfensters liegen.

In Figur 6 sind zwei Beispiele eingezeichnet. Die durchgezogene Linie entspricht dabei dem Ruhezustand eines Patienten und die gestrichelte Linie dem Zustand bei körperlicher Anstrengung. Die körperliche Anstrengung führt zu einem vergrößerten Schlagvolumen und somit zu einer bis auf 12 Ω vergrößerten endsystolischen Impedanz.

Alternativ zum vorstehenden Verfahren kann das Impedanzsignal auch durch Berechnen der ersten und zweiten Ableitungen ausgewertet werden. Die Maximalwerte (möglicherweise innerhalb eines vorgegebenen Zeitfensters) der Ableitung des Kehrwertes der Impedanz korreliert mit der Kontraktilität des Ventrikels.

Zur Bestimmung von belastungsinduzierten Änderungen wird die Signalauswertung im Rahmen einer Langzeitüberwachung separat für den Ruhezustand und den Belastungszustand des Patienten durchgeführt. Auf diese Weise können Langzeitänderungen des Schlagvolumens und des enddiastolischen Volumens oder der Kontraktilität im Ruhezustand und zusätzlich Änderungen der Belastbarkeit ermittelt werden. Ruhezustand und Belastungszustand werden mit Hilfe eines Akzelerometers unterschieden, welcher in einer bevorzugten Ausführungsvariante in das Implantat integriert ist. Das Akzelerometer erzeugt ein der Beschleunigung des Akzelerometers entsprechendes Beschleunigungssignal. Wenn das Beschleunigungssignal als Ausgangssignal des Akzelerometers einen vorgegebenen Schwellwert für eine vorgegebene Zeitspanne überschreitet, wird dies als Belastungszustand gewertet. Wenn die Amplitude des Beschleunigungssignals für eine vorgegebene Zeitspanne unterhalb des Schwellwertes verbleibt, wird dies als Ruhezustand des Patienten gewertet.

Weitere Auswertungen des Signals betreffen Änderungen des enddiastolischen Volumens, des Schlagvolumens, des Ejektionsanteils EF (Ejection fraction) als Quotient aus Schlagvolumen und enddiastolischem Volumen: EF = SV/EDV, oder der Kontraktilität können überwacht werden um den hämodynamischen Zustand eines Patienten zu ermitteln, die Wirkung einer Resynchronisationstherapie oder Medikamentenbehandlung zu beobachten oder bestimmte Betriebsparametereinstellungen eines Implantats zu ermitteln und zu optimieren. Zusätzlich kann der linksventrikuläre Durchmesser beobachtet werden, um beispielsweise Änderungen der Ventrikelabmessungen bei Patienten mit dilatierter oder hypertropher Kardiomyopathie zu ermitteln.

Es sind verschiedene Wege vorgesehen, um den Arzt mit den relevanten Informationen zu versorgen:
1. Homemonitoring:
   Zusammengefasste Impedanzparameter werden mittels einer Homemonitoring-Langstrecken-Telemetrie an ein Servicecenter übermittelt, wo die Daten gespeichert und Trends berechnet werden. Die zusammengefassten Impedanzparameter können beispielsweise Mittelwerte über jeweils 24 h darstellen. Die Datenübertragung kann beispielsweise auf täglicher Basis erfolgen. In dem Servicecenter werden die diagnostischen Daten mit anderen Daten aus dem Implantat kombiniert, zum Beispiel mit der Entwicklung der Herzrate, den Zählerständen verschiedener (Ereignis-) Zähler usw. Die Trends können als Kardio-Reports via Fax oder Internet an einen verantwortlichen Arzt übermittelt und von diesem inspiziert werden. Außerdem können Alarme ausgelöst werden, wenn ein unerwartetes Verhalten der hämodynamischen Werte detektiert wird.
2. Trendaufzeichnungen
   Die aus dem Impedanzsignal extrahierten Parameter werden in dem Implantat als Langzeittrends gespeichert. Diese Trends können beispielsweise anlässlich einer nächstfolgenden Nachsorgeuntersuchung abgefragt und angezeigt werden. Für die abzuspeichernden Trendwerte werden die Impedanzparameter gemittelt, beispielsweise über 24 h, so dass Langzeitänderungen der Hämodynamik zu beobachten sind. Diese Änderungen können beispielsweise aufgrund der Remodellierung in Folge einer Resynchronisationstherapie auftreten.
3. Online-Signalübertragung
   Die Rohdaten des Impedanzsignals und die extrahierten Parameter werden von einem Implantat zu einem externen Gerät, beispielsweise einem Programmiergerät oder einem anderen Datenaufzeichnungsgerät via Telemetrie online übermittelt. Die Daten werden in Echtzeit angezeigt und von dem externen Gerät gespeichert. Der Arzt kann die hämodynamischen Änderungen in Folge bestimmter Interventionen, wie beispielsweise verschiedener Betriebsparametereinstellungen für einen Herzschrittmacher oder Kardioverter/Defibrillator mittels des externen Gerätes beobachten.

Die Auswertung des Impedanzsignals kann außerdem die folgenden Schritte umfassen:
1. Parameteroptimierung
   Verschiedene Betriebsparameter des Implantats können durch Bestimmen des hämodynamischen Zustands optimiert werden. Beispiele hierfür sind die AV-Verzögerungszeit, die VV-Verzögerungszeit oder der Stimulationsmodus eines beispielsweise biventrikulären Schrittmachers. Diese Parameteroptimierung kann interaktiv durch einen Arzt während einer Nachsorgeuntersuchung erfolgen oder automatisch durch das Implantat. Ein Beispiel für eine kontinuierliche, automatische Parameteroptimierung ist die Ratenadaption auf Basis des Schlagvolumens oder der Schlagimpedanz.
2. Tachykardiedetektion oder -diskriminierung
   Im Falle eines implantierbaren Kardioverters/Defibrillators ist die hämodynamische Information wesentlich, um eine ventrikuläre Fibrillation zu bestätigen oder eine ventrikuläre Tachykardie zu detektieren. Es ist insbesondere wichtig, hämodynamisch stabile und hämodynamisch instabile ventrikuläre Tachykardien voneinander zu unterscheiden (zu diskriminieren), um eine unnötige Schockabgabe zu vermeiden. Zu diesem Zweck wird eine Tachykardieepisode zusätzlich durch einen hämodynamischen Sensor ermittelt.

## Patentansprüche

1. Implantat mit Elektrodenleitungsanschlüssen zum Anschluss intrakardialer und/oder epikardialer Elektrodenleitungen, wobei die Elektrodenleitungsanschlüsse zusammen wenigstens drei elektrische Kontakte aufweisen, von denen wenigstens einer einer rechtsventrikulären Elektrode und ein anderer einer linksventrikulären Elektrode zugeordnet ist, mit einer Impedanzbestimmungseinheit (IMP), die eine Strom- oder Spannungsquelle (I) und eine Messeinrichtung (U) für eine entsprechende Spannungs- bzw. Strommessung aufweist, die derart mit den elektrischen Kontakten und gegebenenfalls einer Gehäuseelektrode des Implantats verbunden sind, dass sich eine tri- oder quadrupolare Impedanzmessanordnung ergibt, die ausschließlich ventrikuläre Elektroden und darüber hinaus gegebenenfalls die Gehäuseelektrode umfasst, wobei die Impedanzmessanordnung Impedanzmesswerte liefert und mit einer Auswerteeinheit (EVAL) verbunden ist und die Auswerteeinheit (EVAL) ausgebildet ist, ein Minimum der Impedanzmesswerte innerhalb eines ersten Zeitfensters als enddiastolische Impedanz (EDZ) und ein Maximum der Impedanzmesswerte innerhalb eines zweiten Zeitfensters als endsystolische Impedanz (ESZ) zu ermitteln oder eine endiastolische Leitfähigkeit (EDC) als Kehrwert der enddiastolischen Impedanz (EDZ) und eine ensystolische Leitfähigkeit (ESC) als Kehrwert der endsystolischen Impedanz (ESZ) zu bilden, wobei das erste und das zweite Zeitfenster kürzer als ein jeweiliges Herzzyklusintervall und relativ zu einem ventrikulären Ereignis in einem Herzzyklus orientiert sind, und dass die Auswerteeinheit zusätzlich ausgebildet ist, ein sich aus einem zeitlichen Verlauf der Impedanzmesswerte ergebendes Impedanzsignal zu bestimmen und aus der ersten oder zweiten Ableitung des Impedanzsignals eine eine Kontraktilität eines Herzens entsprechende Kontraktilitätsgröße zu ermitteln, wobei das erste Zeitfenster zu einem ersten Anfangszeitpunkt (x1) vor dem jeweiligen ventrikulären Ereignis beginnt und zu einem ersten Endzeitpunkt (x2) nach diesem ventrikulären Ereignis endet, während das zweite Zeitintervall zu einem zweiten Anfangszeitpunkt (y1) nach jenem ventrikulären Ereignis beginnt und zu einem zweiten Endzeitpunkt (y2) nach dem ventrikulären Ereignis endet.

2. Implantat nach Anspruch 1, **dadurch gekennzeichnet, dass** die Auswerteeinheit (EVAL) ausgebildet ist, aus einer Differenz der enddiastolischen Impedanz (EDZ) und der endsystolischen Impedanz (ESZ) eine ein Schlagvolumen repräsentierende Schlagimpedanz (SZ) zu ermitteln.

3. Implantat nach Anspruch 2, **dadurch gekennzeichnet, dass** die Auswerteeinheit ausgebildet ist, aus der Schlagimpedanz (SZ) und der enddiastolischen Impedanz (EDZ) eine einen Auswurfanteil (EF) repräsentierende EF-Größe zu ermitteln.

4. Implantat nach einem der Anspruch 1 bis 3, **gekennzeichnet durch** einen Speicher (MEM) für Werte für die Schlagimpedanz und/oder die EF-Größe und/oder die Kontraktilität und/oder weitere der Impedanz- und Leitfähigkeitswerte aus den vorangehenden Ansprüchen.

5. Implantat nach Anspruch 4, **dadurch gekennzeichnet, dass** die Auswerteeinheit (EVAL) ausgebildet ist, Werte für die Schlagimpedanz und/oder die EF-Größe und/oder die Kontraktilität zu regelmäßig wiederkehrenden Speicherzeitpunkten zu speichern.

6. Implantat nach Anspruch 5, **dadurch gekennzeichnet, dass** die Auswerteeinheit (EVAL) ausgebildet ist, für einen Zeitraum zwischen zwei aufeinanderfolgenden Speicherzeitpunkten Mittelwerte für die Schlagimpedanz und/oder die EF-Größe und/oder die Kontraktilität zu bilden und den oder die Mittelwerte zu speichern.

7. Implantat nach einem der Ansprüche 1 bis 3, **dadurch gekennzeichnet, dass** die Auswerteeinheit ausgebildet ist, aus der zeitlichen Entwicklung der Schlagimpedanz und/oder der EF-Größe und/oder der Kontraktilität einen Trendwert für die Schlagimpedanz und/oder die EF-Größe und/oder die Kontraktilität zu ermitteln.

8. Implantat nach Anspruch 7, **dadurch gekennzeichnet, dass** die Auswerteeinheit (EVAL) ausgebildet ist, den oder die Trendwerte zu einem jeweiligen Speicherzeitpunkt in den Speicher zu speichern.

9. Implantat nach Anspruch 4, **gekennzeichnet durch** eine Telemetrieeinheit (TEL), die wenigstens einen Telemetriesender umfasst und mit dem Speicher verbunden sowie ausgebildet ist, auf eine Abfrage hin oder zu regelmäßigen Sendezeitpunkten Werte für Schlagimpedanz und/oder die EF-Größe und/oder die Kontraktilität und/oder einen oder mehrere Mittel- oder Trendwerte an ein externes Gerät zu senden.

10. Implantat nach einem der Ansprüche 1 und 9, **gekennzeichnet durch** wenigstens eine Steuer- (CTRL) und eine Stimulationseinheit (STIM), mit der Stimulations-, Kardioversions- und oder Defibrillationsimpulse zu erzeugen und an wenigstens einen der Elektrodenleitungsanschlüsse abzugeben sind.

11. Implantat nach einem der Ansprüche 1 und 10, **gekennzeichnet durch** eine quadrupolare Impedanzmessanordnung, die für den Anschluss an zwei rechtsventrikuläre Elektroden zur Stromeinspeisung und zwei linksventrikuläre, im Koronarsinus angeordnete Elektroden zur Messung der aus dem eingespeisten Strom resultierenden Spannung ausgebildet ist.

## Claims

1. An implant, comprising electrode lead connections for connecting intracardiac and/or epicardial electrode leads, wherein the electrode lead connections together comprise at least three electrical contacts, of which at least one is assigned to a right-ventricular electrode and another is assigned to a left-ventricular electrode, and an impedance determination unit (IMP) comprising a current or voltage source (I) and a measuring device (U) for a corresponding voltage or current measurement, which are connected to the electrical contacts and possibly to a housing electrode of the implant so as to result in a tripolar or quadrupolar impedance measuring system, which includes exclusively ventricular electrodes and in addition possibly the housing electrode, wherein the impedance measuring system supplies impedance measurement values and is connected to an evaluation unit (EVAL), and the evaluation unit (EVAL) is designed to ascertain a minimum of the impedance measurement values within a first time window as the end-diastolic impedance (EDZ) and a maximum of the impedance measurement values within a second time window as the end-systolic impedance (ESZ), or to form an end-diastolic conductivity (EDC) as the reciprocal of the end-diastolic impedance (EDZ) and an end-systolic conductivity (ESC) as the reciprocal of the end-systolic impedance (ESZ), wherein the first and the second time windows are shorter than a respective cardiac cycle interval and oriented relative to a ventricular event in a cardiac cycle, and the evaluation unit is additionally designed to determine an impedance signal resulting from a progression of the impedance measurement values over time and to ascertain a contractility variable representing a contractility of a heart from the first or second derivative of the impedance signal, wherein the first time window begins at a first starting point in time (x1) prior to the respective ventricular event and ends at a first ending point in time (x2) after this ventricular event, while the second time interval begins at a second starting point in time (y1) after this ventricular event and ends at a second ending point in time (y2) after the ventricular event.

2. The implant according to claim 1,
**characterized in that** the evaluation unit (EVAL) is designed to ascertain a stroke impedance (SZ) representing a stroke volume from a difference between the end-diastolic impedance (EDZ) and the end-systolic impedance (ESZ).

3. The implant according to claim 2,
**characterized in that** the evaluation unit is designed to ascertain an EF variable representing an ejection fraction (EF) from the stroke impedance (SZ) and the end-diastolic impedance (EDZ).

4. The implant according to any one of claims 1 to 3,
**characterized by** a memory (MEM) for values for the stroke impedance and/or the EF variable and/or the contractility and/or further impedance and conductivity values from the preceding claims.

5. The implant according to claim 4,
**characterized in that** the evaluation unit (EVAL) is designed to store values for the stroke impedance and/or the EF variable and/or the contractility at regularly recurring storage point in times.

6. The implant according to claim 5,
**characterized in that** the evaluation unit (EVAL) is designed, for a time period between two successive storage point in times, to form mean values for the stroke impedance and/or EF variable and/or the contractility and to store the mean value or values.

7. The implant according to any one of claims 1 to 3,
**characterized in that** the evaluation unit is designed to ascertain a trend value for the stroke impedance and/or the EF variable and/or the contractility from the development of the stroke impedance and/or the EF variable and/or the contractility over time.

8. The implant according to claim 7,
**characterized in that** the evaluation unit (EVAL) is designed to store the trend value or values at a particular storage point in time in the memory.

9. The implant according to claim 4,
**characterized by** a telemetry unit (TEL), which comprises at least one telemetry transmitter and is connected to the memory and which is designed to send values for the stroke impedance and/or the EF variable and/or the contractility and/or one or more mean or trend values to an external unit when prompted or at regular transmission point in times.

10. The implant according to any one of claims 1 and 9,
**characterized by** at least one control unit (CTRL) and one stimulation unit (STIM), by way of which stimulation, cardioversion and/or defibrillation pulses are to be produced and delivered to at least one of the electrode lead connections.

11. The implant according to any one of claims 1 and 10,
**characterized by** a quadrupolar impedance measuring system, which is designed to be connected to two right-ventricular electrodes for current feed and to two left-ventricular electrodes arranged in a coronary sinus for measuring the voltage resulting from the fed current.

## Revendications

1. Implant avec des connexions de lignes d'électrode pour la connexion de lignes d'électrodes intracardiaques et/ou épicardiaques, où les connexions de lignes d'électrode présentent ensemble au moins trois contacts électriques, parmi lesquels au moins un est associé à une électrode du ventricule droit et un autre est associé à une électrode du ventricule gauche, avec une unité de détermination d'impédance (IMP) qui présente une source de courant ou de tension (I) et un dispositif de mesure (U) pour la mesure correspondante d'une tension, respectivement d'un courant, qui est relié avec les contacts électriques et éventuellement une électrode de boîtier de l'implant de telle manière qu'il en résulte un ensemble de mesure d'impédance tri ou quadripolaire qui comprend exclusivement des électrodes ventriculaires et en outre éventuellement l'électrode de boîtier, où l'ensemble de mesure d'impédance délivre des valeurs de mesure d'impédance et est relié avec une unité d'exploitation (EVAL) et l'unité d'exploitation (EVAL) est conçue pour déterminer un minimum des valeurs de mesure d'impédance dans une première fenêtre temporelle sous la forme d'une impédance diastolique finale (EDZ) et un maximum des valeurs de mesure d'impédance dans une deuxième fenêtre temporelle sous la forme d'une impédance systolique finale (ESZ) ou pour constituer une conductivité diastolique finale (EDC) en tant que valeur de reconnaissance de l'impédance diastolique finale (EDZ) et une conductivité systolique finale (ESC) en tant que valeur de reconnaissance de l'impédance systolique finale (ESZ), où les première et deuxième fenêtres temporelles sont plus courtes que l'intervalle du cycle cardiaque correspondant et orientées par rapport à un évènement ventriculaire dans un cycle cardiaque, et que l'unité d'exploitation est en outre conçue pour déterminer un signal d'impédance provenant d'une évolution dans le temps des valeurs de mesure d'impédance et pour déterminer à partir d'une première ou d'une deuxième dérivation du signal d'impédance une taille de contractilité correspondant à une contractilité d'un coeur, où la première fenêtre temporelle commence à un premier instant de début (x1) avant l'évènement ventriculaire en question et termine à un premier instant de fin (x2) après cet évènement ventriculaire, tandis que le deuxième intervalle temporel, commence à un deuxième instant de début (y1) après cet évènement ventriculaire et termine à un deuxième instant de fin (y2) après l'évènement ventriculaire.

2. Implant selon la revendication 1,
**caractérisé en ce que** l'unité d'exploitation (EVAL) est conçue pour déterminer une impédance de battement (SZ) représentant un volume de battement à partir d'une différence entre l'impédance diastolique finale (EDZ) et l'impédance systolique finale (ASZ).

3. Implant selon la revendication 2,
**caractérisé en ce que** l'unité d'exploitation est conçue pour déterminer une grandeur EF représentant une fraction d'éjection (EF) à partir de l'impédance de battement (SZ) et de l'impédance diastolique finale (EDZ).

4. Implant selon l'une des revendications 1 à 3,
**caractérisé par** une mémoire (MEM) pour des valeurs pour l'impédance de battement et/ou la grandeur EF et/ou la contractilité et/ou les autres valeurs d'impédance et de conductivité des revendications précédentes.

5. Implant selon la revendication 4,
**caractérisé en ce que** l'unité d'exploitation (EVAL) est conçue pour stocker des valeurs pour l'impédance de battement et/ou la grandeur EF et/ou la contractilité à des instants de mémorisation se renouvelant régulièrement.

6. Implant selon la revendication 5,
**caractérisé en ce que** l'unité d'exploitation (EVAL) est conçue pour former des valeurs moyennes pour l'impédance de battement et/ou la grandeur EF et/ou la contractilité pour une durée entre deux instants de mémorisation se succédant et pour stocker la ou les valeurs moyennes.

7. Implant selon l'une des revendications 1 à 3,
**caractérisé en ce que** l'unité d'exploitation est conçue pour déterminer une valeur de tendance pour l'impédance de battement et/ou la grandeur EF et/ou la contractilité à partir de l'évolution dans le temps de l'impédance de battement et/ou de la grandeur EF et/ou de la contractilité.

8. Implant selon la revendication 7,
**caractérisé en ce que** l'unité d'exploitation (EVAL) est conçue pour stocker la ou les valeurs de tendance à un instant de mémorisation donné dans la mémoire.

9. Implant selon la revendication 4,
**caractérisé par** une unité de télémétrie (TEL) qui comprend au moins un émetteur de télémétrie et est reliée, ainsi que conçue, avec la mémoire pour émettre des valeurs pour l'impédance de battement et/ou la grandeur EF et/ou la contractilité et/ou une ou plusieurs valeurs moyennes ou de tendance sur demande à des instants d'émission réguliers vers un appareil externe.

10. Implant selon l'une des revendications 1 et 9,
**caractérisé par** au moins une unité de commande (CTRL) et une unité de stimulation (STIM), avec laquelle générer des impulsions de stimulation, de cardioversion et ou de défibrillation et délivrer à au moins une des connexions de ligne d'électrode.

11. Implant selon l'une des revendications 1 à 10,
**caractérisé par** un ensemble de mesure d'impédance quadripolaire qui est conçu pour la connexion à deux électrodes au ventricule droit pour l'alimentation en courant et à deux électrodes au ventricule gauche disposées dans le sinus coronaire pour la mesure de la tension résultant du courant introduit.
